# EUROPEAN PATENT APPLICATION

(11) **EP 1 757 570 A1**
(43) Date of publication of application: **28.02.2007**
(21) Application number: 05018467.0
(22) Date of filing: 25.08.2005
(51) Int. Cl.: C07C 17/395, C07C 19/08

(54) **Process for obtaining a purified hydrofluoroalkane with 2 carbon atoms**

(71) Applicant: Solvay SA, 1050 Brussels (BE)
(72) Inventor: Janssens, Francine, 1800 Vilvoorde (BE); Le Fevere de Ten Hove, Cédric, 5310 Saint Germain (BE)
(74) Representative: Jacques, Philippe

(57) **Abstract**

Hydrofluoroalkanes with 2 carbon atoms comprising unsaturated impurities can be obtained in purified form by contacting them with a inorganic or organic radical initiator such as peroxides or diazo compounds and chlorine.

## Description

The invention relates to a process for obtaining a purified hydrofluoroalkanewith 2 carbon atoms, chosen in particular from 1,1,1-trifluoroethane, pentafluoroethane and especially from 1,1,1,2-tetrafluoroethane.

Hydrofluoroalkanes such as 1,1,1-trifluoroethane /HFC-143a), 1,1,1,2-tetrafluoroethane (HFC-134a) and pentafluoroethane (HFC-125) are composed of carbon, hydrogen and fluorine atoms. They are suitable, i.a., as blowing agents, as refrigerants or as solvents. Highly purified 1,1,2-tetrafluoroethane is also applied as propellant for pharmaceuticals.

Such hydrofluoroalkanes are typically manufactured by reaction of a chloro or chlorofluoro precursor with HF. The crude hydrofluoroalkanes obtained in this reaction often contain impurities such as unconverted reagents, hydrogen fluoride and unsaturated (olefinic) impurities. 1,1,1,2-tetrafluoroethane, for example, typically comprises 2-chloro-1,1-difluoroethylene (HCFC-1122) and /or 1-chloro-2-fluoroethylene (HFC-1131) as impurities. The international patent application WO 9312058 discloses a process for obtaining 1,1,1,2-tetrafluoroethane with a reduced content of HCFC-1122. In this process, crude 1,1,1,2-tetrafluorethane is contacted in the vapor phase with chlorine in the presence of UV light comprising a wave length of 300 to 400 nm providing an exposure of at least about 2 watts-hour per kg of mixture. The reported value for HCFC-1122 after the treatment is considered to be much below 35 wt. ppm.

Object of the present invention was to provide a process for effectively preparing purified C2 alkanes of the general formula C₂HₐF_{b} wherein a is 1, 2 or 3, b is 3, 4 or 5 and the sum of a and b is 6. Another object of the present invention is to provide an integrated process comprising a production step and a purification step for the C2 hydrofluoroalkanes.

These objects are achieved by the process presented in the claims.

The invention relates to a process for obtaining a hydrofluoroalkane with 2 carbon atoms with the general formula C₂HₐF_{b}, wherein a is 1, 2 or 3 and b is 3, 4 or 5 with the proviso that the sum of a and b is 6 with a reduced content of unsaturated organic impurities, by subjecting the hydrofluoroalkane containing unsaturated impurities to at least one purification treatment comprising the contacting with chlorine and an inorganic or organic radical initiator. Preferably, the process is directed to obtain 1,1,1-trifluoroethane, 1,1,1,2-tetrafluoroethane and pentafluoroethane, especially to obtain 1,1,1,2-tetrafluoroethane with a reduced content of unsaturated organic impurities.

The process according to the invention allows an effective reduction of the content of the unsaturated impurities in the hydrofluoroalkanes. The process can be easily performed and can advantageously be integrated, as will be explained below, into the process for preparation of the C2 hydrofluoroalkanes by reaction of chloro or chlorofluoro precursors with HF.

The organic unsaturated impurities which can be removed by the process of the present invention generally comprise 2 to 4 carbon atoms. They may comprise one or more chlorine atoms and one or more fluorine atoms. Their nature depends on the starting material and the reaction conditions of the process for preparing the crude material which has to be purified according to the invention. 1,1,1,2-tetrafluoroethane, when prepared from trichloroethylene and HF, e.g. in a gas phase reaction, typically comprises HCFC-1122 and/or HCFC-1131, as mentioned above. These impurities can be effectively removed. Other potential unsaturated impurities like HFC-1234yf, HFC-1225ye, HFC-1327, HCFC-1122a, CFC-1112a, HCFC-1121 (cis or trans isomer), and HFC-1336 can also be removed by applying the process of the invention.

The treatment with chlorine serves to chlorinate the olefinic impurities in the crude hydrofluoroalkanes, especially those compounds mentioned above. The radical initiator serves to split the chlorine into radicals.

The chlorine treatment process according to the invention can be performed at any stage of the crude product purification whatever its physical state, temperature and pressure. The treatment process according to the invention is preferably carried out in the liquid phase. Though the radical initiator of the present invention can be chosen among inorganic compounds, such as hydrogen peroxide, percarbonates, such as, in particular, sodium percarbonate or perborates, such as sodium perborate, it preferably is an organic compound.. Among peroxide compounds, the ones chosen particularly are diacyl peroxides, peroxydicarbonates, peroxyesters, peroxyketals, ketone peroxides, hydroperoxides and dialkyl peroxides. Diacyl peroxides or peroxydicarbonates are preferably selected among the peroxide compounds. Especially are dilauroyl peroxide, terbutylperoxy-2-ethylhexyl carbonate and very especially dibenzoylperoxide.

Among diazo compounds, 2,2'-azobis(isobutyronitrile) has been found very advantageous.

The radical initiator compound is preferably selected from compounds with a half life from 0.02 to 10 hours at the temperature of the treatment with chlorine. Accordingly, there is a dependency between the selection of a radical initiator and the treatment temperature. There are two choices: if the treatment temperature is given, e.g. by integration of the treatment process into another type of process, e.g. a production process, then the radical initiator is selected such that the half life is in the range given above. Or, if it is desired to use a specific radical initiator, then preferably the treatment with chlorine is performed in a temperature range corresponding to the half life given above. The half life of radical initiators is generally known to the expert. For dibenzoylperoxide (DBP), the half life is for example (in solution in benzene):

| Temperature (°C) | Half life (minutes) |
|---|---|
| 73 | 600 |
| 92 | 60 |
| 131 | 1 |

For 2,2'-azobis(isobutyronitrile), (AIBN), the half life is:

| Temperature (°C) | Half life (minutes) |
|---|---|
| 65 | 600 |
| 82 | 60 |
| 120 | 1 |

The radical initiator is used in an amount providing the desired level of purity of the compound to be treated. Generally, the radical initiator is used in a proportion of at least 10 ppm by weight relative to the hydrofluoroalkane containing impurities. Preferably, at least 20 ppm by weight, more preferably, at least 30 ppm are used. Most frequently, not more than about 10000 ppm by weight of the initiator are used relative to the hydrofluoroalkane containing impurities. Preferably, the amount of the radical initiator does not exceed about 1000 ppm by weight, and more preferably, does not exceed about 300 ppm by weight relative to the hydrofluoroalkane containing impurities.

Alternatively, the amount of radical initiator can be correlated directly to the amount of impurities comprised in the crude hydrofluoroalkane. Generally, the molar ratio between the radical initiator and the unsaturated impurities is equal or greater than 0.01. Generally, it is equal or lower than 10. Preferably, it lies in the range of 0.05 and 5.

The treatment of the crude hydrofluoroalkanes is preferably carried out in the liquid phase. Preferably, chlorine is applied in an excess amount relative to the unsaturated impurities to be chlorinated in the crude hydrofluoroalkane. Generally, chlorine is used in a proportion of 1 or more moles per mole of unsaturated impurities. Preferably, 3 or more moles of chlorine per mole of unsaturated impurities is applied. The upper limit of chlorine is 50 moles per mole unsaturated impurities. Preferably, the amount does not exceed about 15 moles, and more preferably, does not exceed 12 moles chlorine per mole unsaturated impurities. It is advantageous to have all chlorine added reacted so that essentially no chlorine is found in unchanged form after the treatment step. Another advantage is to limit the chlorination of the crude and treated hydrofluoroalkane when observed.

The treatment process of the present invention can be performed at a wide temperature range, which is limited by the desired speed of reactivity at low temperature and the stability of the crude and treated hydrofluoroalkane and resulting pressure at high temperature. The reaction temperature preferably is higher than 20 °C, more preferably higher than 40 °C,. Typically, the temperature will be 150 °C or lower, preferably the temperature of the treatment reaction will be 100 °C or lower. The most preferred range is a temperature between 40 and 100 °C. It has to be noted that higher temperatures allow faster conversion of the impurities.

The treatment with chlorine may be carried out at the autogenous pressure or a higher pressure generated, for example, by introducing a gas such as an inert gas such as nitrogen. In general, the treatment is carried out at a pressure of more than 5 bar (abs.), preferably between 10 and 30 bar (abs.).

The duration of the treatment process may be from about 1 minute to about 30 hours. Preferably, the duration is 10 minutes to 3 hours.

The radical initiator can be introduced into the crude hydrofluoroalkane before the chlorine is introduced. Preferably, the chlorine is introduced into the crude hydrofluoroalkane at a temperature close to the treatment temperature.

The process of the present invention can be applied to any hydrofluoroalkane of the formula mentioned above, irrespective of its origin. For example, one can treat crude hydrofluoroalkanes freshly obtained in a production process, or one can treat hydrofluoroalkanes which have been recycled after use e.g. as refrigerant, one also can treat hydrofluoroalkanes which have already been subjected to a purification step, to further reduce the content of impurities.

A preferred embodiment of the present process for obtaining a hydrofluoroalkane with reduced content of unsaturated impurities provides for a combination with a production process for preparing the crude hydrofluoroalkane. While this combination is applicable to all hydrofluoroalkanes which fall under the formula mentioned above, it is especially suitable for the production and purification of HFC-143a, HFC-134a and HFC-125. This embodiment will be further explained in view of the production and purification of HFC-134a.

It is well known that 1,1,1,2-tetrafluoroethane can be produced from trichloroethylene and HF in the presence of a catalyst. Often, the reaction is performed in two steps. The first step provides for the reaction of trichloroethylene and HF to provide a reaction mixture which comprises 1,1,1-trifluoro-2-chloroethane. This mixture is then further reacted, typically in the gas phase at higher temperatures than the first step, to provide the crude HFC-134a. The crude product comprises HCFC-1122 as main impurities. The crude product leaves the reactor of the gas phase fluorination with a temperature above 300 °C. After the crude product leaves the reactor of the gas phase fluorination, it is subjected to several purification processes performed in the gas or in the liquid phase. In a preferred embodiment, the treatment process of the invention is performed where the crude product is in the liquid phase. For example, the temperature of the crude product at these locations is comprised between 40 and 100°C.

In this preferred embodiment of the invention which combines production and purification steps to form an integrated process, the temperature of the crude reaction product may vary in dependence of the point of the production plant where the crude product is drafted for the chlorine treatment. If the temperature differs from the temperature at which the treatment with chlorine shall be performed, it can be adjusted by cooling or heating the crude product. As described above, either a radical initiator is selected with a suitable half life range at the predetermined treatment temperature, or the temperature is adjusted to adapt it to a predetermined radical initiator to shift the half life of this initiator into the desired range.

After having performed the treatment step of the present invention, the hydrofluoroalkane will generally be separated from the impurities which are mostly hydrochloro-fluoroalkanes, unconverted radical initiator and reaction products of the latter. Most easily, the separation is performed by a distillation using one or more columns. The columns which may be used are known per se. For example, one may use conventional plate columns or dual-flow plate columns or columns with bulk or structured packaging. The number of theoretical plates is generally at least 5, preferably at least 10, more preferably at least 20.

The treatment process of the present invention can be applied to hydrofluoroalkanes the content of impurities of which can vary in a broad range. For example, 1,1,1,2-tetrafluoroethane can be treated with a content of HCFC-1122 and/or HCFC-1131 between 10 ppm or even less up to 5000 ppm and more.

By the treatment process of the invention, the content of the unsaturated impurities, such as HCFC-1122 in 1,1,1,2-tetrafluoroethane, can be reduced to a very low level, down to 4 or even 2 ppm which fulfils the specification for pharmaceutical applications, e.g. as propellant for aerosols.

The following examples are intended to describe the invention further without delimiting it.

### Examples

### General procedure:

Examples 1.1 and 1.2: A predetermined amount of HCFC-1122 was added to 1,1,1,2-tetrafluoroethane to evaluate the effectivity of the treatment process at high concentrations of unsaturated impurities. The treatment was performed in in a Teflon or glass coated ~160ml reactor with magnetic stirrer. After the indicated reaction time, , the reactor is cooled down to about 40°C and a sample is taken from the liquid phase for analysis by chromatography.

Examples 2.1 to 2.3 were performed with crude HFC-134a from a production plant. The amount of HCFC-1122 is low even in the crude product.

### Example 1: Purification of 1,1,1,2-tetrafluoroethane comprising high amounts of HCFC-1122

### 1.1. Use of Azobisisobutyronitrile (AIBN) as radical initiator:

1,1,1,2-tetrafluoroethane with an added content of 3400 ppm of HCFC-1122 was filled in the autoclave and AIBN (1000 wt ppm) and chlorine (2.3g) was added thereto. The ratio Cl2/HCFC-1122 was 10 mol/mol, the ratio AIBN/HCFC-1122 was 0.2 mol/mol. The autoclave was heated to 80 °C and kept at that temperature for 0.5 h. The autoclave then was cooled, a sample from the liquid phase taken and analyzed. The content of HCFC-1122 in this sample has been found to be 7 ppm.

The contents of the reactor were heated for a further 0.5 h. A sample from the liquid phase was taken and analyzed, and it was found that HCFC-1122 was no longer detectable.

### 1.2. Use of dilauroyl peroxide (LP) as radical initiator:

Example 1.1 was repeated, but this time, dilauroyl peroxide (1000 ppm by weight) was used. The ratio Cl₂/HCFC-1122 was 11 mol/mol, the ratio AIBN/HCFC-1122 was 0.07 mol/mol.

The reactor was heated to 100 °C, and samples of the liquid phase were taken after 0.5 h, 1 h and 2 h and analyzed by chromatography. The sample taken after 0.5 h contained 14 ppm, the sample taken after 1 hour contained 8 ppm. In the sample taken after 2 h, no HCFC-1122 was detectable.

### Example 2: Purification of 1,1,1,2-tetrafluoroethane comprising low amounts of HCFC-1122

### 2.1. Use of azobisisobutyronitrile (AIBN) as radical initiator:

The crude 1,1,1,2-tetrafluoroethane contained HCFC-1122 in an amount of 32 ppm. AIBN and chlorine was added so that the crude product contained 164 weight-ppm AIBN, and 724 weight-ppm chlorine. Accordingly, the molar ratio of chlorine to HCFC-1122 was 38, the molar ratio of AIBN to HCFC-1122 was 3.7, and the molar ratio of chlorine to AIBN was 10.2.

The treatment was performed at a temperature of 75 °C. After 76 minutes, a sample of the liquid phase was analyzed by chromatography. The content of HCFC-1122 was reduced to 3.3 ppm.

### 2.2. Use of AIBN as radical initiator with prolonged treatment time

Example 2.1 was repeated, but the treatment time was 2 hours and 6 minutes. A sample taken from the liquid phase had a residual HCFC-1122 content of 2 ppm.

### 2.3. Use of dibenzoyl peroxide (DBP) as radical initiator:

The crude 1,1,1,2-tetrafluoroethane contained HCFC-1122 in an amount of 32 ppm. DBP and chlorine was added so that the crude product contained 332 weight-ppm DBPand 675 weight-ppm chlorine. Accordingly, the molar ratio of chlorine to HCFC-1122 was 35.5, the molar ratio of AIBN to HCFC-1122 was 3.4, and the molar ratio of chlorine to AIBN was 13.9.

The treatment was performed at a temperature of 75 °C. After 26 minutes, a sample of the liquid phase was analyzed by chromatography. The content of HCFC-1122 was reduced to 4.2 ppm.

The examples demonstrate that unsaturated impurities even in very low concentration can be transformed very effectively to chlorinated products which then can be separated easily from the hydrofluoroalkane by distillation.

## Claims

1. Process for obtaining a hydrofluoroalkane with 2 carbon atoms with the general formula C₂HₐF_{b}, wherein a is 1, 2 or 3 and b is 3, 4 or 5 with the proviso that the sum of a and b is 6, preferably for obtaining 1,1,1 -trifluoroethane, 1,1,1,2-tetrafluoroethane and pentafluoroethane with a reduced content of unsaturated organic impurities, by subjecting the hydrofluoroalkane containing unsaturated impurities to at least one purification treatment comprising the contacting with chlorine and an inorganic or organic radical initiator.

2. Process according to claim 1, wherein the treatment with chlorine and the initiator is performed at a temperature between 20 and 100 °C.

3. Process according to claim 1wherein the treatment is performed at a pressure between 10 and 25 bar (abs.).

4. Process according to claim 1 wherein the molar ratio between the radical initiator and the unsaturated impurities is in the range of 0.01 to 10.

5. Process according to claim 4 wherein the ratio between the radical initiator and the unsaturated impurities is in the range of 0.05 to 5.

6. Process according to claim 1 wherein the molar ratio between chlorine and the unsaturated impurities is 1 and 50.

7. Process according to claim 1 wherein the treatment is carried out in the liquid phase.

8. Process according to claim 1 wherein the radical initiator is an organic radical initiator selected from peroxide compounds and diazo compounds.

9. Process according to claim 1 wherein 1,1,1,2-tetrafluoroethane is treated which comprises 1-chloro-2,2-difluoroethylene as an impurity.

10. Process according to claim 1 wherein 1,1,1,2-tetrafluoroethane is treated which comprises 1-fluoro-2-chloroethylene as an impurity.

11. Process according to claim 1 wherein 1,1,1,2-tetrafluoroethane is treated which was produced in gas phase reaction from trichloroethylene and HF .

12. Process according to claim 1 which is integrated into a process for preparing hydrofluorocarbons with 2 carbon atoms from chlorinated precursors and HF, optionally in the presence of a catalyst.

13. Process according to claim 12 which is a gas phase reaction.

14. Process according to claim 1 wherein the hydrofluoroalkane is liquid and at a temperature in the range of 40 to 100 °C when it is treated, and the radical initiator is selected so that its half life at the treatment temperature is in the range of 1 minute to 5 hours.

15. Process according to claim 11 wherein the radical initiator is dibenzoyl peroxide, azo-bis-isobutyronitrile or terbutylperoxy-2-ethylhexyl carbonate.

16. Process according to claim 1 wherein the treated hydrofluoroalkane is isolated by distillation.

17. Process according to claim 1 wherein the hydrofluoroalkane after distillation is obtained in pharma grade quality.
